# EUROPEAN PATENT APPLICATION

(11) **EP 2 105 137 A1**
(43) Date of publication of application: **30.09.2009**
(21) Application number: 09004106.2
(22) Date of filing: 23.03.2009
(51) Int. Cl.: A61K 31/727, A61K 38/55, A61P 9/10

(54) **A novel use of bivalirudin in the treatment of acute coronary syndrome**

(30) Priority: 27.03.2008 CA 2623449
(71) Applicant: The Medicines Company, Parsippany NJ 07054 (US)
(72) Inventor: Baldo, Lance, West New York, NJ 07093 (US); Plent, Stephanie, Winchester, MA 01890 (US); Skerjanec, Simona, Pittstown, NJ 08867 (US); Meanwell, Clive, Bernardsville, NJ 07924 (US)
(74) Representative: Bösl, Raphael Konrad

(57) **Abstract**

A method of treating acute coronary syndrome (ACS) in a patient, comprising administering a therapeutically effective amount of an indirect thrombin inhibitor (unfractionated heparin UFH or low molecular weight heparin LMWH such as enoxaparin) to the patient, and subsequently administering a therapeutically effective amount of a direct thrombin inhibitor, namely bivalirudin, to the patient wherein the direct thrombin inhibitor is administered prior to and during a surgical procedure.

## Description

### FIELD OF THE INVENTION

The invention relates to the use of bivalirudin in the treatment of patients with acute coronary syndrome (ACS). More particularly, it relates to the use of bivalirudin to treat ACS comprising the administration of a therapeutically effective amount of an indirect thrombin inhibitor and the subsequent administration of a therapeutically effective amount of a direct thrombin inhibitor.

### BACKGROUND OF THE INVENTION

Acute vascular disease, such as myocardial infarction, stroke, pulmonary embolism, deep vein thrombosis, peripheral arterial occlusion, and other blood system thromboses constitute major health risks. Such diseases are caused by either partial or total occlusion of a blood vessel by a blood thrombus, which contains fibrin and platelets.
Combination of potent antithrombotic and antiplatelet agents, although effective in suppressing ischemic adverse events related to percutaneous intervention (PCI), also result in high rates of hemorrhagic complications, the occurrence of which have been strongly associated with early and late mortality.
Bivalirudin is a direct-acting synthetic antithrombin that is much more active against clot-bound thrombin than either unfractionated heparin (UFH) or low molecular weight heparin (LMWH). The drug does not activate platelets, bind to plasma proteins, or cause heparin-induced thrombocytopenia (HIT) and it has linear pharmacokinetics with a short half-life of 25 minutes.
Optimal management for moderate and high risk patients with non-ST elevation acute coronary syndromes (NSTE-ACS) includes an invasive strategy with intensive antithrombin therapy.¹ Most patients with acute coronary syndromes are currently treated with either UFH or enoxaparin initiated either in the emergency department or in a transferring hospital prior to coronary intervention. In recent clinical trials, such as the Superior Yield of the New Strategy of Enoxaparin, Revascularization and Glycoprotein IIb/IIIa Inhibitors (SYNERGY)² and the Organization to Assess Strategies for Ischemic Syndromes (OASIS-5),³ 72% and 50% of patients respectively received clinician-selected antithrombin treatment before randomization.
Transitions from an upstream heparin to an alternate antithrombin, however, have been associated with an increase in adverse clinical outcomes. In SYNERGY, patients who crossed over after randomization from UFH to enoxaparin or vice versa had increased rates of death/myocardial infarction (MI) within 30 days compared to those who did not cross over (22.0% vs. 14.2%, UFH; 17.4% vs. 13.5%, enoxaparin). The rate of transfusion doubled with crossover (35% vs. 15.1%, UFH; 30.2% vs. 15.3%, enoxaparin)⁴. However, because these crossovers occurred after randomization, association or causality cannot be defined. Patients may have crossed over due to ischemia or bleeding or, alternatively, crossing over may have caused an ischemic or bleeding event.² Consistent therapy, however, was associated with lower rates of death/MI.⁵ Consistent antithrombin therapy, therefore, has been recommended in guidelines while changing therapy has been discouraged.⁶
The prior art demonstrates that, in the treatment of ACS comprising the administration of heparin followed by the administration of bivalirudin, the anti-coagulants can be switched at the time of PCI. For example, the REPLACE-2 study¹³ evaluated switching from LMWH or UFH to bivalirudin and found that there was no effect of the duration of antithrombin discontinuation on bleeding for patients who had received UFH or LMWH and who were subsequently randomized to receive bivalirudin. The results in this study, however, related only to patients undergoing urgent or elective PCI. The investigators found that switching from enoxaparin to bivalirudin among patients undergoing PCI was not associated with an increase in major bleeding, regardless of the duration from last enoxaparin administration to PCI.
In the Phase III Bivalirudin Angioplasty Trial (BAT), the investigators found that a conversion between bivalirudin and heparin (each at respective therapeutic doses) resulted in maintenance of safe and adequate anticoagulation. Again, however, bivalirudin was not administered prior to PCI.
The SWITCH study found that switching from enoxaparin to bivalirudin for patients with ACS undergoing PCI appeared to be clinically safe without increased risk of major bleeding complications, regardless of the time of enoxaparin administration. The investigators compared 3 groups of patients: those that had received their last enoxaparin dose prior to PCI in 0-4 hours, 4-8 hours and 8-12 hours. All patients received a full dose of bivalirudin during PCI. It was found that in patients with ACS who received enoxaparin pretreatment, switching to full-dose bivalirudin during subsequent PCI is clinically safe and feasible, regardless of when the last enoxaparin dose was administered.
The Acute Catheterization and Urgent Intervention Triage strategY (ACUITY)⁷ trial demonstrated superior net clinical outcomes with similar rates of ischemia and significantly less major bleeding with bivalirudin monotherapy compared to UFH/enoxaparin plus a GP IIb/IIIa inhibitor.
The ACUITY trial demonstrated that, in patients with moderate and high risk NSTE-ACS, treatment with bivalirudin monotherapy resulted in similar rates of composite ischemia, a 47% relative (2.7% absolute) reduction in major bleeding, and improved net clinical outcomes compared with UFH/enoxaparin plus a GP IIb/IIIa inhibitor. In light of recent data indicating an association between bleeding and mortality,^{8,9} these results suggest that bivalirudin is an attractive alternative for patients with NSTE-ACS.⁸
Current guidelines recommend that anticoagulants not be switched prior to PCI. For example, the European Society of Cardiology guidelines for the diagnosis and treatment of NSTE-ACS recommend:
"At PCI procedures, the initial anticoagulant should also be maintained during the procedure regardless of whether this treatment is UFH (I-C), enoxaparin (IIa-B),
or bivalirudin (I-B)..."
   The present invention, however, demonstrates that switching from UFH or enoxaparin to bivalirudin monotherapy prior to PCI results in comparable ischemic outcomes and an approximately 50% reduction in major bleeding.

### SUMMARY OF THE INVENTION

The present invention includes a method to switch patients receiving an indirect thrombin inhibitor to a direct thrombin inhibitor prior to PCI to reduce bleeding events and improve net clinical outcomes.
The present invention includes a method of treating patients with ACS wherein the patients receive an initial dose of an indirect thrombin inhibitor followed by a dose of a direct thrombin inhibitor prior to PCI. The applicant has, for the first time, demonstrated a benefit to the switch before any surgical procedure or intervention.
The invention includes a method of treating ACS in a patient, comprising the administration of a therapeutically effective amount of an indirect thrombin inhibitor and the subsequent administration of a therapeutically effective amount of a direct thrombin inhibitor, wherein the administration of the direct thrombin inhibitor is prior to or at the time of angiography and subsequently during a surgical procedure.
The invention also includes a method of reducing incidents of bleeding, reducing the need for blood transfusions and reducing ischemic events during the treatment of ACS, comprising the administration of a therapeutically effective amount of an indirect thrombin inhibitor followed by the administration of a therapeutically effective amount of a direct thrombin inhibitor, wherein the administration of the direct thrombin inhibitor is prior to or at the time of angiography and subsequently during a surgical procedure.
The invention also provides a method of reducing major bleeding events, reducing the need for blood transfusions and reducing ischemic events in a patient with ACS comprising administering a therapeutically effective amount of an indirect thrombin inhibitor and administering a therapeutically effective amount of a direct thrombin inhibitor, wherein both the indirect thrombin inhibitor and direct thrombin inhibitor are administered prior to or at the time of angiography and subsequently during a surgical procedure.

### BRIEF DESCRIPTION OF THE FIGURES

Figure 1: Enrollment and randomization to consistent therapy, either UFH or enoxaparin, or switch (at the time of randomization) to bivalirudin from either UFH or enoxaparin. Naïve patients are those who received no antithrombin therapy prior to randomization. (UFH=unfractionated heparin, Enox=enoxaparin)

Figure 2: Comparison of patients randomized to bivalirudin versus UFH/enoxaparin, by prior antithrombin therapy.

### DETAILED DESCRIPTION

The present invention relates to a method of treating acute coronary syndrome (ACS) in a patient, comprising the administration of a therapeutically effective amount of an indirect thrombin inhibitor and the subsequent administration of a therapeutically effective amount of a direct thrombin inhibitor, wherein the administration of the direct thrombin inhibitor is prior to a surgical procedure or intervention.
The applicant has demonstrated that switching from UFH or enoxaparin to bivalirudin monotherapy prior to PCI resulted in an approximately 50% reduction in major bleeding as compared to maintaining the UFH or enoxaparin therapy. Patients with ACS in whom an invasive strategy is planned can be safely switched from UFH or enoxaparin to bivalirudin monotherapy prior to or at the time of angiography or other procedure.
This finding is important in light of the previous studies which have shown transitions from an upstream heparin to an alternate antithrombin to be associated with an increase in adverse clinical outcomes such as ischemia or increased bleeding whereas consistent therapy, i.e. non-switching, has been associated with lower rates of death/MI.
Switching before PCI is important because there is a preference in clinical practice to delay cardiac catheterization if a patient has been administered LMWH. Furthermore, bivalirudin, via its specific mode of action, may, especially in the context of ACS, add to the anticoagulant properties of enoxaparin, but unlike UFH, without an associated increase in bleeding risk. This effect appears to be independent of the time and bioavailability of the last enoxaparin dose which, due to the difficulty of determining when the last dose of enoxaparin is given in typical clinical practice, might be of specific clinical relevance. Additionally, switching to bivalirudin permits greater flexibility to move to a surgical intervention if the angiogram reveals that cardiac bypass surgery (CABG) surgery is indicated. The shorter half-life of bivalirudin is desirable in those patients who may need to go to surgery because it permits more precise control of the anticoagulation therapy.
The present invention demonstrates that patients with ACS in whom an invasive strategy is planned can be safely switched from UFH or enoxaparin to bivalirudin monotherapy, prior to angiography. This approach results in an approximate 50% reduction in major bleeding with similar rates of ischemia. The demonstration that net clinical outcomes are improved by switching patients pre-treated with UFH or enoxaparin to bivalirudin is clinically relevant, since moderate and high risk NSTE-ACS patients are often treated with either UFH or enoxaparin in the emergency department or at a transferring hospital.
The safety and efficacy of switching from prior UFH or enoxaparin to bivalirudin may be partly explained by the mechanism of action of the antithrombin agents. Bivalirudin directly targets thrombin, specifically inhibits both clot bound and fluid phase thrombin and has a short half-life of approximately 25 minutes. Bivalirudin becomes biologically inactive when it is cleaved by thrombin and then dissociates, allowing thrombin to return to normal hemostatic activity.¹⁴ In contrast, UFH and enoxaparin are non-specific, indirect thrombin inhibitors with relatively longer half-lives. Combining two agents such as UFH and enoxaparin may lead to excessive bleeding if there is an additive effect of two agents with prolonged anti-Xa activity. Adding bivalirudin with its lack of Xa activity to UFH or enoxaparin would not increase the existing anti-Xa activity, and any incremental anti-IIa effect would be temporary given its short half-life and rapid clearance.
In patients with moderate and high risk NSTE-ACS, switching from either UFH or enoxaparin to bivalirudin monotherapy prior to angiography results in a similar rate of composite ischemia compared with consistent treatment with UFH or enoxaparin plus a GP IIb/IIIa inhibitor. Further, switching to bivalirudin monotherapy results in an approximate 50% reduction in major bleeding compared to remaining on either UFH or enoxaparin in the overall population, as well as in high risk patients and in those managed with PCI. Patients naïve to antithrombin therapy who are administered bivalirudin monotherapy have a significant reduction in major bleeding with similar rates of composite ischemia compared with patients administered UFH or enoxaparin plus a GP IIb/IIIa inhibitor.
The switch to bivalirudin monotherapy can be initiated up to 72 hours prior to angiography. Preferably, angiography occurs within 48 hours of the switch to bivalirudin, but the switch can occur as late as 0-30 minutes prior to angiography.

### SPECIFIC EMBODIMENTS

1. Use of a direct thrombin inhibitor to treat acute coronary syndrome (ACS) in a patient, comprising:
   first administration of a therapeutically effective amount of an indirect thrombin
   inhibitor to the patient, and subsequent administration of a therapeutically effective amount of the direct
   thrombin inhibitor to the patient;
   wherein the direct thrombin inhibitor is administered prior to or at the time of angiography and subsequently during a surgical procedure.
2. The use as specified under No. 1, wherein the indirect thrombin inhibitor is unfractionated heparin (UFH) or low molecular weight heparin (LMWH).
3. The use as specified under No. 1, wherein the direct thrombin inhibitor is reversible.
4. The use of as specified under No. 3, wherein the direct thrombin inhibitor is bivalirudin.
5. The use of as specified under No. 1, wherein the surgical procedure is percutaneous coronary intervention (PCI), percutaneous transluminal coronary angioplasty (PTCA), cardiac bypass surgery (CABG), or a PCI/CABG hybrid procedure.
6. The use of as specified under No. 5, wherein administration of the direct thrombin inhibitor to the patient is prior to or at the time of angiography with a bolus of 0.1 mg/kg followed by an infusion at a rate of 0.25 mg/kg/hr and during the surgical procedure with a bolus of 0.5 mg/kg followed by an infusion at a rate of 1.75 mg/kg/hr.
6. The use of as specified under No. 1, wherein the direct thrombin inhibitor exhibits no anti-Xa activity.
7. The use of as specified under No. 1, wherein the direct thrombin inhibitor exhibits a shorter half-life than the indirect thrombin inhibitor.
9. The use of as specified under No. 1, wherein the direct thrombin inhibitor therapy reduces the risk of bleeding during the surgical procedure.
10. The use of claim 1, wherein the direct thrombin inhibitor therapy reduces the risk of requiring blood transfusions during the treatment of the ACS.
11. The use of as specified under No. 1, wherein the direct thrombin inhibitor therapy reduces the risk of ischemic events during the treatment of the ACS.
12. The use as specified under Nos. 9 to 11, wherein the patient is at risk of heparin-induced thrombocytopenia or thrombosis syndrome (HIT/HITTS).
13. Use of bivalirudin to treat acute coronary syndrome (ACS) in a patient, comprising:
   first administration of a therapeutically effective amount of an indirect thrombin inhibitor to the patient, and
   subsequent administration of a therapeutically effective amount of bivalirudin to the patient;
wherein the bivalirudin is administered prior to or at the time of angiography and subsequently during a surgical procedure.

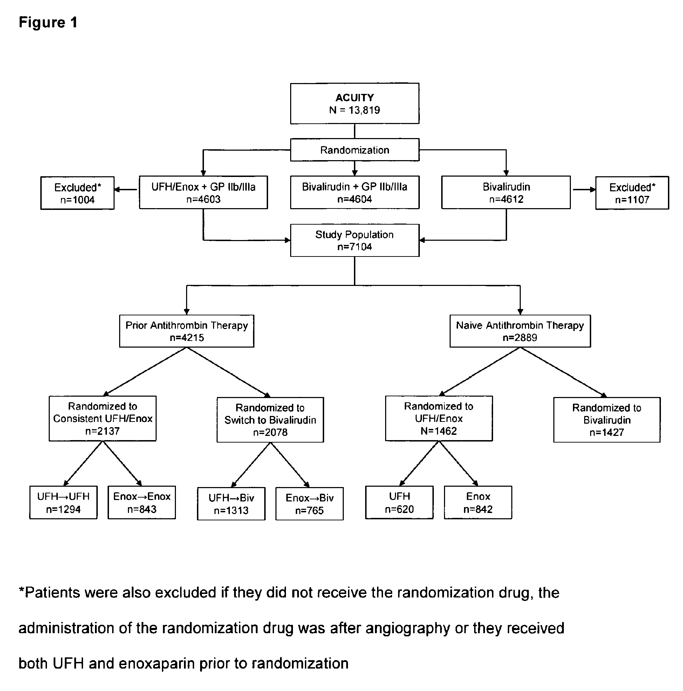

Example A total of 4215 patients received prior antithrombin therapy with either UFH or enoxaparin before randomization. Of these, 2137 were randomized to receive the same antithrombin plus a GP IIb/IIIa inhibitor (consistent), while 2078 patients were randomized to receive bivalirudin (switch). There were 2889 patients naïve to antithrombin therapy at randomization, and of these, 1462 patients were randomized to UFH/enoxaparin plus a GP IIb/IIIa inhibitor and 1427 to bivalirudin monotherapy (Figure 1).

### Patients receiving prior antithrombin therapy

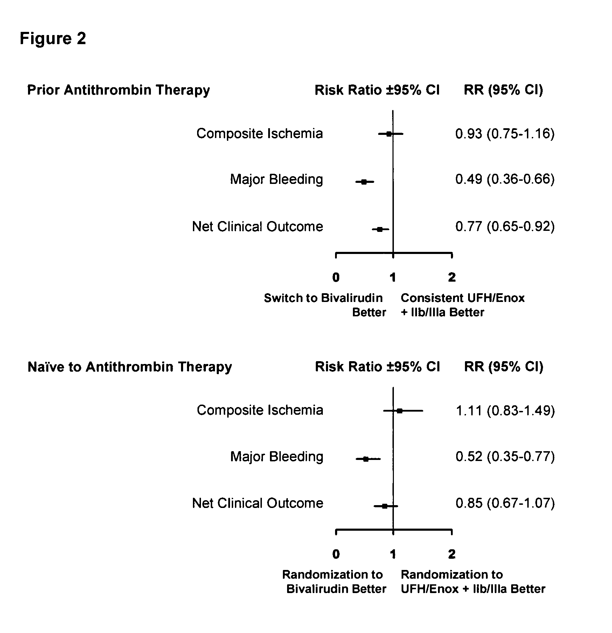

As shown in Table 1, patients randomized to consistent UFH/enoxaparin therapy were on median 1 year older than patients switched to bivalirudin, though more patients switched to bivalirudin had high risk features (defined as elevated cardiac biomarkers or ECG changes at presentation); there were no other significant baseline demographic differences. At 30 days, there was no difference in composite ischemia between the two groups: 6.9% for patients switched to bivalirudin vs. 7.4% for patients remaining on consistent UFH/enoxaparin ([RR 0.93; 0.75-1.16], p=0.52). Major bleeding was significantly reduced by 51%: 2.8% for patients switched to bivalirudin vs. 5.8% for patients remaining on consistent UFH/enoxaparin ([RR 0.49; 0.36-0.66], p<0.01). Transfusions were also lower in the patients switched to bivalirudin vs. patients remaining on consistent UFH/enoxaparin (1.5% vs. 2.6% [RR 0.60; 0.39-0.92], p=0.02). (Table 2, Figure 2). In patients defined as high risk and in patients undergoing PCI, composite ischemia was similar but bleeding was significantly lower in patients switched to bivalirudin (Table 2).

### Patients naive to prior antithrombin therapy

As shown in Table 1, patients naïve to antithrombin therapy randomized to UFH/enoxaparin plus a IIb/IIIa inhibitor or to bivalirudin had similar baseline characteristics except there were more patients with a history of prior MI and prior PCI in the bivalirudin group. Composite ischemia occurred with similar frequency in the two groups, while major bleeding was significantly lower with bivalirudin (Table 2, Figure 2).

### Comparison of outcomes in patients receiving prior antithrombin therapy to antithrombin naive patients

Results of formal interaction testing indicated that there was no interdependency between prior antithrombin therapy, randomized treatment assignment and outcome. The interaction p-values for patients randomized to bivalirudin or UFH/enoxaparin plus a IIb/IIIa inhibitor and prior antithrombin therapy were not significant (composite ischemia, p= 0.34; non-CABG major bleeding, p = 0.80; net clinical outcomes p=0.51).

**Table 1: Baseline characteristics**

| | Consistent Therapy UFH/Enoxaparin n = 2137 | Switch to Bivalirudin n = 2078 | P1 | Naïve UFH/Enoxaparin n = 1462 | Naïve Bivalirudin n = 1427 | P2 |
|---|---|---|---|---|---|---|
| Age (median [range], yrs) | 63.0 [23,91] | 62 [20,92] | 0.03 | 63.0 [30,91] | 63.0 [25,92] | 0.69 |
| Male | 1538 (72.0%) | 1459 (70.2%) | 0.21 | 973(66.6%) | 943 (66.1%) | 0.79 |
| Weight (median [IQR], kg) | 84 [73,96] | 84 [73,96] | 0.22 | 84.0 [72,95] | 84.0 [73,95] | 0.49 |
| Diabetes | 595/2122 (28.0%) | 536/2063 (26.0%) | 0.13 | 432/1453 (29.7%) | 430/1416 (30.4%) | 0.71 |
| Hypertension | 1391/2134 (65.2%) | 1337/2070 (64.6%) | 0.69 | 1013/1453 (69.7%) | 1008/1425 (70.7%) | 0.55 |
| Hyperlipidemia | 1175/2095 (56.1%) | 1131/2034 (55.6%) | 0.76 | 889/1446 (61.5%) | 895/1410 (63.5%) | 0.27 |
| Current smoker | 631/2104 (30.0%) | 635/2051 (31.0%) | 0.50 | 384/1437 (26.7%) | 372/1405 (26.5%) | 0.88 |
| Prior MI | 668/2096 (31.9%) | 627/2033 (30.8%) | 0.48 | 455/1424 (32.0%) | 496/1388 (35.7%) | 0.03 |
| Prior PCI | 799/2126 (37.6%) | 770/2062 (37.3%) | 0.87 | 675/1453(46.5%) | 712/1414 (50.4%) | 0.04 |
| Prior CABG | 402/2135 (18.8%) | 382/2074 (18.4%) | 0.73 | 292/1458 (20.0%) | 300/1423 (21.1%) | 0.48 |
| Thienopyridine exposure | 1327/2108 (63.0%) | 1342/2072 (64.8%) | 0.22 | 927/1443 (64.2%) | 914/1404 (65.1%) | 0.63 |
| Renal insufficiency* | 396/2016 (19.6%) | 344/1989 (17.3%) | 0.06 | 247/1376(18.0%) | 268/1338 (20.0%) | 0.17 |
| US | 1180/2137 (55.2%) | 1180/2078 (56.8%) | 0.30 | 982/1462 (67.2%) | 965/1427 (67.6%) | 0.79 |
| High Risk** | 1581/2047 (77.2%) | 1496/2024 (73.9%) | 0.01 | 805/1345(59.9%) | 818/1322 (61.9%) | 0.28 |
| Troponin elevation | 1235/1902 (64.9%) | 1188/1883 (63.1%) | 0.24 | 486/1172 (41.5%) | 507/1126 (45.0%) | 0.09 |
| ST-segment deviation | 748/2136 (35.0%) | 669/2078 (32.2%) | 0.05 | 453/1461 (31.0%) | 428/1426 (30.0%) | 0.56 |
| TIMI risk score | | | 0.11 | | | 0.23 |
| - 0-2 | 310/1879 (16.5%) | 340/1845 (18.4%) | | 192/1272 (15.1%) | 157/1232 (12.7%) | |
| - 3-4 | 990/1879 (52.7%) | 987/1845 (53.5%) | | 731/1272 (57.5%) | 721/1232 (58.5%) | |
| - 5-7 | 579/1879 (30.8%) | 518/1845 (28.1%) | | 349/1272 (27.4%) | 354/1232 (28.7%) | |

| | | | | | | |
|---|---|---|---|---|---|---|
| *Calculated creatinine clearance using the Cockcroft-Gault equation <60 ml/min; **Elevated cardiac biomarkers or ST-segment deviation ≥ 1mm; IQR = interquartile range; MI=myocardial infarction; PCI=percutaneous coronary intervention; CABG=coronary artery bypass surgery P1 = P-Value for Comparison for Switch to Bivalirudin vs. Consistent Therapy UFH/Enoxaparin P2 = P-Value for Comparison for Naïve Bivalirudin vs. Naïve UFH/Enoxaparin | | | | | | |

**Table 2: Clinical outcomes at 30 days**

| | Consistent | Switch | | | | Naïve | Naïve | | | |
|---|---|---|---|---|---|---|---|---|---|---|
| | UFH/Enoxaparin | Bivalirudin | RR | CI | P | UFH/Enoxaparin | Bivalirudin | RR | CI | P |
| | n=2137 | n=2078 | | | | n=1462 | n=1427 | | | |
| Composite | 159(7.4%) | 144(6.9%) | 0.93 | 0.75-1.16 | 0.52 | 81(5.5%) | 88(6.2%) | 1.11 | 0.83-1.49 | 0.47 |
| Ischemia | | | | | | | | | | |
| Death | 27(1.3%) | 21(1.0%) | 0.80 | 0.45-1.41 | 0.44 | 12(0.8%) | 14(1.0%) | 1.20 | 0.55-2.58 | 0.65 |
| MI | 117(5.5%) | 100(4.8%) | 0.88 | 0.68-1.14 | 0.33 | 51(3.5%) | 67(4.7%) | 1.35 | 0.94-1.92 | 0.10 |
| Unplanned | 37(1.7%) | 47(2.3%) | 1.31 | 0.85-2.00 | 0.22 | 35(2.4%) | 28(2.0%) | 0.82 | 0.50-1.34 | 0.43 |
| revasc | | | | | | | | | | |
| Major | 124(5.8%) | 59(2.8%) | 0.49 | 0.36-0.66 | <0.01 | 71(4.9%) | 36(2.5%) | 0.52 | 0.35-0.77 | <0.01 |
| bleeding | | | | | | | | | | |
| Transfusion | 55(2.6%) | 32(1.5%) | 0.60 | 0.39-0.92 | 0.02 | 35 (2.4%) | 15 (1.1%) | 0.44 | 0.24 - 0.80 | <0.01 |
| Net clinical | 255(11.9%) | 191(9.2%) | 0.77 | 0.65-0.92 | <0.01 | 138(9.4%) | 114(8.0%) | 0.85 | 0.67-1.07 | 0.17 |
| outcome | | | | | | | | | | |
| **High Risk*** | **n=1581** | **n=1496** | | | | **n=805** | **n=818** | | | |
| Ischemic | 129(8.2%) | 115(7.7%) | 0.94 | 0.74-1.20 | 0.63 | 54 (6.7%) | 62 (7.6%) | 1.13 | 0.79 - 1.61 | 0.50 |
| Composite | | | | | | | | | | |
| Death | 26(1.6%) | 20(1.3%) | 0.81 | 0.46-1.45 | 0.48 | 9(1.1%) | 9(1.1%) | 0.98 | 0.39-2.47 | 0.97 |
| MI | 97(6.1%) | 84(5.6%) | 0.92 | 0.69-1.22 | 0.54 | 37(4.6%) | 50(6.1%) | 1.33 | 0.88-2.01 | 0.18 |
| Unplanned | 25(1.6%) | 33(2.2%) | 1.40 | 0.83-2.33 | 0.21 | 20(2.5%) | 18(2.2%) | 0.89 | 0.47-1.66 | 0.71 |
| revasc | | | | | | | | | | |
| Major | 103(6.5%) | 53(3.5%) | 0.54 | 0.39-0.75 | <0.01 | 46(5.7%) | 25(3.1%) | 0.53 | 0.33-0.86 | 0.01 |
| bleeding | | | | | | | | | | |
| Transfusion | 47(3.0%) | 30(2.0%) | 0.67 | 0.43-1.06 | 0.09 | 21(2.6%) | 8(1.0%) | 0.37 | 0.17-0.84 | 0.02 |
| Net clinical | 206(13.0%) | 159(10.6%) | 0.82 | 0.67-0.99 | 0.04 | 89 (11.1%) | 80(9.8%) | 0.88 | 0.66-1.18 | 0.40 |
| outcome | | | | | | | | | | |
| **PCI** | **n=1236** | **n=1292** | | | | **n=808** | **n=831** | | | |
| Ischemic | 101(8.2%) | 116(9.0%) | 1.10 | 0.85-1.42 | 0.47 | 54(6.7%) | 57(6.9%) | 1.03 | 0.72-1.47 | 0.89 |
| composite | | | | | | | | | | |
| Death | 8(0.6%) | 13(1.0%) | 1.55 | 0.65-3.74 | 0.32 | 4(0.5%) | 3(0.4%) | 0.73 | 0.16-3.25 | 0.68 |
| MI | 78(6.3%) | 84(6.5%) | 1.03 | 0.76-1.39 | 0.84 | 35(4.3%) | 50(6.0%) | 1.39 | 0.91-2.12 | 0.13 |
| Unplanned | 30(2.4%) | 39(3.0%) | 1.24 | 0.78-1.99 | 0.36 | 24(3.0%) | 21(2.5%) | 0.85 | 0.48-1.52 | 0.58 |
| revasc | | | | | | | | | | |
| Major | 83(6.7%) | 45(3.5%) | 0.52 | 0.36-0.74 | <0.01 | 47(5.8%) | 22(2.6%) | 0.46 | 0.28-0.75 | <0.01 |
| bleeding | | | | | | | | | | |
| Transfusion | 35(2.8%) | 23(1.8%) | 0.63 | 0.37-1.06 | 0.08 | 21(2.6%) | 8(1.0%) | 0.37 | 0.17-0.83 | 0.02 |
| Net clinical | 163(13.2%) | 153(11.8%) | 0.90 | 0.73-1.10 | 0.31 | 91(11.3%) | 75(9.0%) | 0.80 | 0.60-1.07 | 0.13 |
| outcome | | | | | | | | | | |

| | | | | | | | | | | |
|---|---|---|---|---|---|---|---|---|---|---|
| *High risk=elevated cardiac biomarkers or ST-segment deviation ≥1mm; RR=risk reduction: CI=confidence interval; Revasc=revascularization; PCI=percutaneous coronary intervention; MI=myocardial infarction | | | | | | | | | | |

### Comparisons of consistent therapy with UFH and consistent therapy with enoxaparin to switch to bivalirudin

Tables 3a and 3b show clinical outcomes according to type of heparin therapy (UFH or enoxaparin) in patients on prior antithrombin therapy and in patients naive to antithrombin therapy. In patients switched from either UFH or enoxaparin to bivalirudin, there were similar rates of composite ischemia and significant reductions in major bleeding; in patients naive to antithrombin therapy, there were similar rates of composite ischemia and significant reductions in major bleeding for those randomized to bivalirudin vs. either UFH or enoxaparin plus a GP IIb/IIIa inhibitor.

**Table 3a Clinical outcomes at 30 days according to consistent therapy on UFH vs switch from UFH to bivalirudin**

| | | Prior Antithrombin Therapy | | | |
|---|---|---|---|---|---|
| | Consistent UFH | Switch Bivalirudin | | | |
| | | | RR | CI | P |
| | n=1294 | n=1313 | | | |
| Composite Ischemia | 99(7.7%) | 97(7.4%) | 0.97 | 0.74-1.26 | 0.80 |
| Major bleeding | 82(6.3%) | 37(2.8%) | 0.44 | 0.30-0.65 | <0.01 |
| Transfusion | 36(2.8%) | 23(1.8%) | 0.63 | 0.38-1.06 | 0.08 |
| Net clinical outcome | 162(12.5%) | 123(9.4%) | 0.75 | 0.60-0.93 | 0.01 |
| UFH=Unfractionated heparin; RR=Risk reduction; CI=Confidence internal | | | | | |

| | | Naive to Antithrombin Therapy | | | |
|---|---|---|---|---|---|
| | Randomized to UFH | Randomized to Bivalirudin | | | |
| | | | RR | CI | P |
| | n=620 | n=1427 | | | |
| Composite Ischemia | 32(5.2%) | 88(6.2%) | 1.19 | (0.81-1.77) | 0.38 |
| Major bleeding | 29(4.7%) | 36(2.5%) | 0.54 | (0.33-0.87) | 0.01 |
| Transfusion | 16(2.6%) | 15(1.1%) | 0.41 | (0.20-0.82) | 0.01 |
| Net clinical outcome | 57(9.2%) | 114(8.0%) | 0.87 | (0.64-1.18) | 0.36 |

**Table 3b: Clinical outcomes at 30 days according to consistent therapy on enoxaparin vs switch from enoxaparin to bivalirudin**

| | | Prior Antithrombin Therapy | | | |
|---|---|---|---|---|---|
| | Consistent Enoxaparin n=843 | Switch Bivalirudin n=765 | RR | CI | P |
| Composite Ischemia | 60(7.1%) | 47(6.1%) | 0.86 | 0.60-1.25 | 0.43 |
| Major bleeding | 42(5.0%) | 22 (2.9%) | 0.58 | 0.35-0.96 | 0.03 |
| Transfusion | 19(2.3%) | 9(1.2%) | 0.52 | 0.24-1.15 | 0.11 |
| Net clinical outcome | 93(11.0%) | 68(8.9%) | 0.81 | 0.60-1.08 | 0.15 |
| RR=risk reduction; CI=confidence interval | | | | | |

| | | Naive to Antithrombin | Therapy | | |
|---|---|---|---|---|---|
| | Randomized to Enoxaparin | Randomized to Bivalirudin | RR | CI | P |
| | | | | | |
| | n=842 | n=1427 | | | |
| Composite Ischemia | 49(5.8%) | 88(6.2%) | 1.06 | (0.76-1.49) | 0.74 |
| Major bleeding | 42(5.0%) | 36(2.5%) | 0.51 | (0.33-0.78) | <0.01 |
| Transfusion | 19(2.3%) | 15(1.1%) | 0.47 | (0.24-0.91) | 0.03 |
| Net clinical outcome | 81 (9.6%) | 114(8.0%) | 0.83 | (0.63-1.09) | 0.18 |

### REFERENCES

1. Anderson JL, et al., ACC/AHA 2007 Guidelines for the Management of Patients With Unstable Angina/Non-ST-Elevation Myocardial Infarction: A Report of the American College of Cardiology/American Heart Association Task Force on Practice Guidelines (Writing Committee to Revise the 2002 Guidelines for the Management of Patients With Unstable Angina/Non-ST-Elevation Myocardial Infarction) Developed in Collaboration with the American College of Emergency Physicians, the Society for Cardiovascular Angiography and Interventions, and the Society of Thoracic Surgeons Endorsed by the American Association of Cardiovascular and Pulmonary Rehabilitation and the Society for Academic Emergency Medicine. J. Am. Coll. Cardiol. 2007;50(7):e1-e157.
2. Mahaffey KW, et al., High-risk patients with acute coronary syndromes treated with low-molecular-weight or unfractionated heparin: outcomes at 6 months and 1 year in the SYNERGY trial. JAMA. 2005;294:2594-2600.
3. Yusuf S, et al., Comparison of fondaparinux and enoxaparin in acute coronary syndromes. N Engl J Med. 2006;354:1464-1476.
4. Ferguson JJ, et al., Enoxaparin vs unfractionated heparin in high-risk patients with non-ST-segment elevation acute coronary syndromes managed with an intended early invasive strategy: primary results of the SYNERGY randomized trial. JAMA. 2004;292:45-54.
5. Cohen M, et al., on behalf of the SYNERGY Trial Investigators. A subgroup analysis of the impact of prerandomization antithrombin therapy on outcomes in the SYNERGY trial: enoxaparin versus unfractionated heparin in non-ST-segment elevation acute coronary syndromes. J Am Coll Cardiol. 2006;48:1346-1354.
6. Bassand JP, et al., Guidelines for the diagnosis and treatment of non-ST-segment elevation acute coronary syndromes: The Task Force for the Diagnosis and Treatment of Non-ST-Segment Elevation Acute Coronary Syndromes of the European Society of Cardiology. Eur Heart J. 2007;28:1598-1660.
7. Stone GW, et al., for the ACUITY Investigators. Bivalirudin for patients with acute coronary syndromes. N Engl J Med. 2006;355:2203-2216.
8. Manoukian SV, et al., Impact of major bleeding on 30-day mortality and clinical outcomes in patients with acute coronary syndromes: an analysis from the ACUITY Trial. J Am Call Cardiol. 2007;49:1362-1368.
9. Eikelboom JW, et al., Adverse impact of bleeding on prognosis in patients with acute coronary syndromes. Circulation. 2006;114:774-782.
10. Stone GW, et al., Acute Catheterization and Urgent Intervention Triage strategY (ACUITY) trial: study design and rationale. Am Heart J. 2004;148:764-775.
11. Antman EM, et al., The TIMI risk score for unstable angina/non-ST elevation MI: a method for prognostication and therapeutic decision making. JAMA. 2000;284:835-842.
12. White HD, et al., Efficacy and safety of enoxaparin compared with unfractionated heparin in high-risk patients with non-ST-segment elevation acute coronary syndrome undergoing percutaneous coronary intervention in the Superior Yield of the New Strategy of Enoxaparin, Revascularization and Glycoprotein IIb/IIIa Inhibitors (SYNERGY) trial. Am Heart J. 2006;152:1042-1050.
13. Gibson CM, et al., Association of Prerandomization Anticoagulant Switching With Bleeding in the Setting of Percutaneous Coronary Intervention (A REPLACE-2 Analysis). Am J Cardiol. 2007;99:1687-1690.
14. Bates SM and Weitz JI. Direct thrombin inhibitors for treatment of arterial thrombosis: potential differences between bivalirudin and hirudin. Am J Cardiol. 1998;82:12P-18P.
15. Bassand JP, et al. Guidelines for the diagnosis and treatment of non-ST-segment elevation acute coronary syndromes. Eur. Heart J. 2007;28:1598-1660.

## Claims

1. A direct thrombin inhibitor for use in a method for the treatment of acute coronary syndrome (ACS) in a patient, comprising:
a therapeutically effective amount of an indirect thrombin inhibitor for an administration to the patient, and
a therapeutically effective amount of the direct thrombin inhibitor for a subsequent administration to the patient;
wherein the direct thrombin inhibitor is bivalirudin,
wherein the indirect thrombin inhibitor is unfractionated heparin (UFH) or low molecular weight heparin (LMWH) and
wherein the administration of the direct thrombin inhibitor is prior to or at the time of angiography and subsequently during a surgical procedure.

2. The thrombin inhibitor of claim 1, wherein the surgical procedure is percutaneous coronary Intervention (PCI), percutaneous transluminal coronary angioplasty (PTCA), cardiac bypass surgery (CABG), or a PCI/CABG hybrid procedure.

3. The thrombin inhibitor of claim 2, wherein the direct thrombin inhibitor administration is prior to or at the time of angiography with a bolus of 0.1 mg/kg followed by an infusion at a rate of 0.25 mg/kg/hr and during the surgical procedure with a bolus of 0.5 mg/kg followed by an infusion at a rate of 1.75 mg/kg/hr.

4. The thrombin inhibitor of claim 1, wherein the direct thrombin inhibitor exhibits a shorter half-life than the indirect thrombin inhibitor.

5. The thrombin inhibitor of claim 1, wherein the direct thrombin inhibitor therapy reduces the risk of bleeding during the surgical procedure.

6. The thrombin inhibitor of Claim 1, wherein the direct thrombin Inhibitor therapy reduces the risk of requiring blood transfusions during the treatment of the ACS.

7. The thrombin inhibitor of Claim 1, wherein the direct thrombin inhibitor therapy reduces the risk of ischemic events during the treatment of the ACS.

8. The thrombin inhibitor of any one of Claims 5 to 7, wherein the patient is at risk of heparin-induced thrombocytopenia or thrombosis syndrome (HIT/HITTS).

9. A direct thrombin inhibitor for use in a method for the treatment of acute coronary syndrome (ACS) in a patient who has previously received an administration of a therapeutically effective amount of an indirect thrombin inhibitor, the use comprising:
a therapeutically effective amount of the direct thrombin inhibitor for subsequent administration to the patient;
wherein the direct thrombin inhibitor is bivalirudin,
wherein the indirect thrombin inhibitor is unfractionated heparin (UFH) or low molecular weight heparin (LMWH) and
wherein the administration of the direct thrombin inhibitor is prior to or at the time of angiography and subsequently during a surgical procedure.

10. The thrombin inhibitor of claim 9, wherein the surgical procedure is percutaneous coronary Intervention (PCI), percutaneous transluminal coronary angioplasty (PTCA), cardiac bypass surgery (CABG), or a PCI/CABG hybrid procedure.

11. The thrombin inhibitor of claim 10, wherein the direct thrombin inhibitor administration is prior to or at the time of angiography with a bolus of 0.1 mg/kg followed by an infusion at a rate of 0.25 mg/kg/hr and during the surgical procedure with a bolus of 0.5 mg/kg followed by an infusion at a rate of 1.75 mg/kg/hr.

12. The thrombin inhibitor of claim 9, wherein the direct thrombin Inhibitor exhibits a shorter half-life than the indirect thrombin Inhibitor.

13. The thrombin inhibitor of Claim 9, wherein the direct thrombin Inhibitor therapy reduces the risk of bleeding during the surgical procedure.

14. The thrombin inhibitor of claim 9, wherein the direct thrombin inhibitor therapy reduces the risk of requiring blood transfusions during the treatment of the ACS.

15. The thrombin inhibitor of claim 9, wherein the direct thrombin inhibitor therapy reduces the risk of ischemic events during the treatment of the ACS.

16. The thrombin inhibitor of any one of claims 13 to 15, wherein the patient is at risk of heparin-induced thrombocytopenia or thrombosis syndrome (HIT/HITTS).
